# FASCICULE DE BREVET EUROPEEN

(11) **EP 0 511 903 B1**
(45) Date de publication et mention de la délivrance du brevet: **14.10.1998**
(21) Numéro de dépôt: 92401164.6
(22) Date de dépôt: 23.04.1992
(51) Int. Cl.: C08G 65/32, A61K 47/48, C07K 17/08

(54) **Conjugué de calcitonine et de polyéthylène glycol**
Konjugate von Calcitonin und Polyethylenglycol
Conjugate of calcitonin and polyethylene glycol

(30) Priorité: 23.04.1991 FR 9104990
(43) Date de publication de la demande: 04.11.1992
(73) Titulaire: TEVA PHARMACEUTICAL INDUSTRIES LTD., Jerusalem (IL)
(72) Inventeur: Wantier, Henri, B-7370 Elouges (BE); Mathieu, Fabienne, B-1400 Nivelles (BE); Baudrihaye, Marc, B-1410 Waterloo (BE); De Clercq, Pierre, B-9000 Gand (BE)
(74) Mandataire: Warcoin, Jacques

(56) Documents cités:
- EP-A- 0 400 472
- EP-A- 0 400 486
- US-A- 4 179 337
- PATENT ABSTRACTS OF JAPAN, vol. 10, no. 390 (C-394)(2447), 26 décembre 1986; & JP-A-61 178 926 (TAKEDA CHEM. IND. LTD) 11-08-1986

## Description

La présente invention concerne un conjugué de Calcitonine ou un de ses analogues avec du polyéthylène glycol par couplage covalent, et un procédé de préparation de ce conjugué.

Dans la présente demande on entend par "Calcitonine" (CT) l'une quelconque des différentes calcitonines naturelles notamment la calcitonine humaine, de saumon ou d'anguille. Et "par analogue de la Calcitonine" on entend un analogue biologique actif d'une des calcitonines naturelles ayant une activité de même nature et possédant une séquence d'acides aminés substantiellement identique à celle d'une des calcitonines naturelles.

La Calcitonine est une hormone peptidique régulant l'homéostasie calcique et exerçant des effets biologiques sur l'os, le rein, le système gastro intestinal et le système nerveux central. Elle est utilisée en thérapeutique dans plusieurs situations pathologiques dont l'ostéoporose, l'hypercalcémie, la maladie de Paget, la pancréatite aiguë et l'atrophie de Sudeck. Aucune méthode ne permettant actuellement l'administration orale dans des conditions satisfaisantes, elle est administrée par voie parentérale (injections IM, SC, IV et récemment spray nasal). Plusieurs types de calcitonines (CT) sont utilisés, notamment la CT humaine synthétique, la CT de saumon synthétique, la CT d'anguille ou un analogue synthétique et la CT naturelle de porc.

Certains analogues biologiquement actifs et ayant une activité de même nature que celle des calcitonines ont été décrits possédant substantiellement les mêmes séquences d'acides aminés que les calcitonines naturelles (voir entre autres celles des références l à 8). Il s'agit en général de polypeptides reproduisant la même séquence qu'une calcitonine naturelle avec éventuellement une ou deux délétion(s) et/ou dont quelques acides aminés sont modifiés par rapport à cette calcitonine naturelle ou de polypeptides composés par l'assemblage de portions de différentes calcitonines naturelles éventuellement modifiées comme indiqué ci-dessus.

Les séquences des différentes calcitonines naturelles ont été décrites (références 1 à 10).

L'injection de CT hétérologue à l'homme (saumon, anguille, porc,...) provoque régulièrement l'apparition d'anticorps anticalcitonines. Ceux-ci peuvent être très préjudiciables dans certaines circonstances. La CT humaine homologue n'induit habituellement pas la formation d'anticorps. Cependant, l'activité spécifique de la CT humaine est environ 40 fois inférieure à celle de la CT de saumon. Cette différence d'activité, qui implique l'utilisation de doses beaucoup plus élevées de ce coûteux produit pour obtenir le même effet thérapeutique, est attribuée à plusieurs facteurs et notamment :
- une plus grande résistance à la dégradation enzymatique de la CT de saumon par rapport à la CT humaine (référence 11)
- une demi-vie plasmatique prolongée pour la CT de saumon par rapport à la CT humaine (référence 12)
- une meilleure affinité de la CT de saumon pour le récepteur membranaire spécifique par rapport à la CT humaine (référence 13).

Le but de la présente invention est de fournir une forme de calcitonine
- dont l'immunogénicité et/ou l'antigénicité est réduite de manière à éviter les effets préjudiciables liés à la réponse immunitaire, par exemple une Calcitonine de type saumon à haute activité spécifique mais dépourvue d'inconvénients liés à la sensibilisation ;
- et/ou dont l'activité spécifique serait augmentée en raison (a) d'une affinité pour le récepteur conservée ou accrue et (b) d'une biodisponibilité améliorée notamment par augmentation de la demi vie plasmatique et/ou de la résistance à la dégradation enzymatique.

Des conjugués de polyéthylène glycol et de protéines ont été décrits dans la littérature (références 13,14). Ces conjugués présentent les caractéristiques suivantes :
- une réduction de l'immunogénicité et l'antigénicité de la protéine
- une augmentation de la demi-vie plasmatique de la protéine
- éventuellement une augmentation de la résistance à la dégradation enzymatique
- éventuellement une conservation des propriétés biologiques de la protéine.

Les conjugués décrits comportent généralement des protéines ou des polypeptides de haut poids moléculaires (≥ 10.000) sur lesquels sont couplés un grand nombre de polymères de polyéthylène glycol sur différents sites des protéines (au moins 3 et généralement plus de 10), le plus souvent par réaction avec les groupes aminés des lysines (ou des arginines) qui sont soit totalement substituées soit le plus souvent partiellement substituées de manière aléatoire aboutissant à un produit final hétérogène.

On a observé selon l'invention que le couplage de polyéthylène glycol à des polypeptides de petits poids moléculaires (≤ 10.000) pose des problèmes très différents de celui à des polypeptides macromoléculaires. D'une part, le couplage d'un polyéthylène glycol de P.M. 5000 (par exemple) influence fortement les propriétés biologiques d'un peptide de taille équivalente ou inférieure (par exemple la Calcitonine qui a un poids moléculaire de 3500), et ceci a fortiori si plusieurs polymères de polyéthylène glycol sont couplés. En d'autres termes, il s'ensuit une perte de l'activité biologique, par exemple par réduction de l'affinité au récepteur dans le cas d'un peptide ligand. D'autre part, le nombre de polymères de polyéthylène glycol ou la densité de polymères de polyéthylène glycol nécessaire pour obtenir les caractéristiques attendues ne sont pas non plus connues dans la littérature. Il n'est donc pas possible de prévoir si un, deux, trois polymère de polyéthylène glycol ou plus, couplés aux polypeptides de petits poids moléculaires permettent de reproduire les qualités obtenues avec un grand nombre de polymères de polyéthylène glycol sur des peptides macromoléculaires.

Les couplages sont en général effectués sur les fonctions NH₂ des polypeptides parce que ce couplage peut être réalisé par une approche directe ne nécessitant pas la protection d'autres groupes réactionnels. En dehors de sa simplicité, cette technique présente également un moindre risque d'entraîner une modification structurelle des polypeptides macromoléculaires au cours du procédé.

Des méthodes de couplage de polyéthylène glycol sélectives adaptées à d'autres sites du polypeptide, n'ont pas été développées avant la présente invention.

Or on a découvert selon l'invention, que le site de couplage a une importance prédominante sur l'effet obtenu.

Dans le cas particulier de la Calcitonine, un conjugué avec du polyéthylène glycol est déjà décrit dans le brevet EP0400472A2. Il s'agit d'un conjugué de polyéthylène glycol et de Calcitonine où deux molécules de polyéthylène glycol greffées sur un "spacer" possédant une fonction carboxyle ou autre susceptible de réagir avec une fonction amine, sont ajoutées par couplage dudit "spacer" sur chacune des fonctions amine réactionnelles de la calcitonine d'anguille via ladite fonction réactive du "spacer". Une augmentation de demi-vie plasmatique de ce produit est décrite mais l'affinité pour le récepteur n'est pas présentée. Les inventeurs ont reproduit un couplage d'un polyéthylène glycol sur les mêmes fonctions aminés réactionnelles de la calcitonine et confirmé l'augmentation de demi vie plasmatique. Cependant, ce produit a perdu sa capacité de liaison au récepteur membranaire de la calcitonine, du moins de manière significative (< 0,5 % par rapport à la CT intacte, cf. exemple 2) et ne présente plus d'activité hypocalcémiante in vivo comparable (cf. exemple 5), ce qui confirme la perte ou la réduction drastique d'activité. D'une manière plus générale, cela démontre aussi l'impossible extrapolation des données obtenues avec les protéines macromoléculaires de grande taille à des petits polypeptides spécifiques.

La présente invention fournit un conjugué de Calcitonine et de polyéthylène glycol, caractérisé par le couplage du polyéthylène glycol sur un groupe carboxyle libre de la Calcitonine.

Plus précisément, la présente invention a pour objet un conjugué de Calcitonine ou un polypeptide analogue avec un ou plusieurs composé(s) polymérique(s) comportant chacun un ou plusieurs polymère(s) de polyéthylène glycol, par lien covalent du ou desdits composé(s) polymériques avec respectivement un ou au moins un groupe carboxyle de la Calcitonine ou son analogue.

Les calcitonines possèdent en effet au moins un groupe carboxyle libre, en général en leur acide aminé en position 15. Il s'agit soit d'un acide aspartique, en particulier dans la calcitonine humaine, de rat, de boeuf ou de mouton, soit d'un acide glutamique en particulier dans la calcitonine de saumon ou celle d'anguille et leurs analogues. La calcitonine de porc possède un acide aminé acide unique en position en position 30 (30-Glu). Certaines Calcitonines possèdent deux voire trois acides aminés acides. Ainsi les calcitonines d'anguille et de poulet en possèdent deux en position 15 et 26 (15-Glu et 26-Asp), les calcitonines de bovin et de mouton en possèdent également deux en position 15 et 30 (15-Asp et 30-Glu).

Le couplage sur une fonction carboxylique résulte en un couplage spécifique et reproductible du composé polymérique dans une ou deux position(s) toujours identique(s) et spécifique(s) du polypeptide.

Lorsque le couplage se fait sur les fonctions NH₂ des acides aminés Lys et Arg de la Calcitonine, ceci entraîne le couplage de polymères de polyéthylène glycol en des positions différentes de la calcitonine et surtout peut masquer des épitopes biologiquement actifs, compte tenu de la localisation et de la fonction des acides aminés Lys et Arg dans les calcitonines.

Contrairement aux produits cités plus haut, la Calcitonine conjuguée avec du polyéthylène glycol en position 15 conserve une affinité de liaison avec le récepteur comparable à la CT non modifiée (exemple 3) et une activité hypocalcémiante in vivo également semblable (exemple 6). Elle présente également l'avantage d'une demi-vie plasmatique prolongée (exemple 4) ainsi que le masquage sélectif d'un épitope antigénique, le couplage étant réalisé dans un site immunologiquement réactif sans interférer avec le site biologiquement actif (exemple 5).

Plus généralement la présente invention a donc pour objet un conjugué de Calcitonine ou un polypeptide analogue possédant substantiellement la même séquence d'acides aminés qu'une Calcitonine naturelle,et notamment au moins un acide aminé acide en particulier en position l5, et une activité biologique de même nature qu'une Calcitonine naturelle, avec un ou plusieurs composé(s) polymérique(s) comportant chacun un ou plusieurs polymère(s) de polyéthylène glycol, par lien covalent du ou desdits composé(s) avec le ou les groupe(s) carboxyle(s) de la Calcitonine ou son analogue en des positions qui n'altèrent pas négativement son activité biologique, notamment un groupe carboxyle d'un acide aminé en position 15 de la Calcitonine ou son analogue.

Le couplage du composé polymérique sur la ou les fonction(s) carboxyle(s) accessible(s) de la Calcitonine ou son analogue n'exclut pas nécessairement son couplage sur une autre fonction accessible dont le couplage sur cette position n'altère pas négativement l'activité biologique de la CT ou son analogue.

Dans un mode de réalisation donc le composé polymérique est couplé sur le groupe carboxyle accessible en position 15 de la Calcitonine ou son analogue sans nécessairement exclure le couplage d'un autre composé polymérique sur un autre carboxyle ou une autre fonction accessible de la Calcitonine ou son analogue.

Le composé polymérique à base de polyéthylène glycol peut être couplé à cette fonction carboxyle de la Calcitonine ou son analogue, via une fonction chimique réactive susceptible de se coupler de façon stable à une fonction carboxyle libre de la Calcitonine ou son analogue.

De préférence le couplage covalent consiste en un lien peptidique amide -CONH- entre ledit groupe carboxyle de la Calcitonine ou son analogue et une fonction NH₂ dudit composé polymérique comportant un ou plusieurs polymères de polyéthylène glycol, mais bien entendu d'autres types de lien chimique peuvent convenir.

Ladite fonction NH₂ peut être greffée directement sur un unique polyéthylène glycol de préférence à une de ses extrémités ou elle peut être portée par un groupement chimique faisant office de bras de liaison ("linker ou spacer") entre le ou les polymère(s) de polyéthylène glycol et la Calcitonine ou son analogue. Ce bras de liaison peut notamment consister en un radical hydrocarboné linéaire ou cyclique dans lequel un ou plusieurs CH₂ peuvent être remplacés par O, N ou S, tel qu'un alkyl, un aryl ou un hétérocycle, sur lequel est ou sont greffé(s) le ou les notamment 2 à 4 polymères de polyéthylène glycol, en général à une de leurs extrémités.

Dans ce cas, ledit composé polymérique est constitué par ledit bras de liaison portant une fonction NH₂, et le ou lesdits polymère(s) de polyéthylène glycol dont la fonction hydroxyle à leur extrémité terminale libre est de préférence substituée par un groupe alkyl tel que méthyl de manière à donner un groupe terminal alcoxy, tel que méthoxy.

Des dérivés de polyéthylène glycol portant un ou plusieurs groupe(s) NH₂ ont été décrits dans la littérature ou peuvent être obtenus d'une manière générale par des procédés conventionnels bien connus de l'homme de l'art (voir notamment références 15 à 18).

Ainsi, des dérivés de polyéthylène glycol appropriés sont par exemple l'α-amino-ω-méthylpoly(oxyéthylène) de formule NH₂(CH₂CH₂O)ₙ CH₃ (ou α(2-aminoéthyl)-ω-méthoxypoly(oxyéthylène) de formule :
CH₃O-[CH₂CH₂O]ₙ-CH₂CH₂NH₂) où le groupe amine est directement greffé sur le polyéthylène glycol ou un dérivé avec un bras intermédiaire tel que NH₂-(CH₂)_{n'}-NH-CO-O-CH₂-CH₂-O(CH₂CH₂O)ₙCH₃. Ces composés sont disponibles dans le commerce. D'une manière générale, comme on l'a déjà mentionné, on peut utiliser tout composé comprenant un ou plusieurs polymère(s) de polyéthylène glycol et une fonction amine libre pour le couplage au groupe carboxyle de la Calcitonine ou son analogue, et plus particulièrement un polyéthylène glycol homopolymère possédant d'une part un groupe NH₂ terminal à une extrémité, soit directement greffé, soit relié par un bras, et d'autre part un groupe OH terminal à l'autre extrémité qui est de préférence substitué avec un groupe alkyl tel que méthyl pour donner un groupe alcoxy de préférence méthoxy.

L'exemple 1 décrit une voie de synthèse de l'amino-méthylpoly(oxyéthylène) mentionné ci-dessus ; toutefois, d'autres voies de synthèses de ce composé ont été décrites (notamment référence 15).

Dans les formules ci-dessus n et n' représentent des nombres entiers variables suivant le poids moléculaire du polymère concerné.

Le conjugué comprend de préférence un ou plusieurs polymère(s) de polyéthylène glycol, ou poly(oxyéthylène) de poids moléculaire entre 500 et 20.000, de préférence de 4.000 à 10.000.

La présente invention a également pour objet un procédé de préparation d'un conjugué selon l'invention, caractérisé en ce que l'on effectue un couplage peptidique entre la fonction carboxyle de la calcitonine ou d'un de ses analogues, et un groupe NH₂ d'un composé comportant un ou plusieurs polymères de polyéthylène glycol, par un lien amide. L'étape de couplage proprement dite s'effectue sur une fonction carboxyle de la CT qui n'est plus libre mais activée par un groupe activateur de fonction acide conventionnel ; ceci implique également la protection précédente des fonctions aminés de la CT également par un groupe protecteur conventionnel, tel que le groupe fmoc.

Dans un mode de réalisation la Calcitonine ou son analogue possède un unique acide aminé acide (aspartique ou glutamique) en position 15. Il peut donc s'agir par exemple d'une calcitonine de saumon ou d'anguille ou un analogue avec un acide glutamique en position 15 de sa séquence d'acides aminés, ou une calcitonine humaine ou un analogue avec un acide aspartique en position 15 de sa séquence ou acides aminés. La substitution d'un acide glutamique par un acide aspartique ou vice versa est évidemment possible dans un analogue synthétique de ces Calcitonines.

D'autres caractéristiques et avantages de la présente invention apparaitront à la lumière des exemples qui vont suivre.

### Exemple 1

### Synthèse de l'α-amino-ω-méthylpoly(oxyéthylène) ou α(2-aminoéthyl)-ω-méthoxypoly(oxyéthylène)

(1) Une solution de 200 g de monométhylpoly(oxyéthylène) 5.000 [CH₃-(OCH₂CH₂)ₙ-OH] dans 1,4 litre de toluène est chauffée à reflux et concentrée. Après distillation de ca 800 ml de toluène, le toluène résiduel est écarté d'abord sous vide moyen (16 mm Hg) ensuite sous haut vide (0,2 mm Hg ).
   On ajoute au résidu 400 ml de pyridine, 200 mg de diméthyl-aminopyridine (DMAP) et 450 ml de dichlorométhane. A la solution, refroidie à 0°C est ajouté sur une période de 20 mn 38 g de chlorure de p-toluènesulfonyle dans 160 ml de dichlorométhane. On agite durant 16 heures durant lesquelles la température remonte à 12°C.
   On refroidit ensuite le mélange à 0°C, on y ajoute 30 g de glace et après avoir agité durant 30 minutes on déverse le mélange sur une solution de 550 ml de HCl (2 Mol) et 1 kg de glace. On extrait avec 200 ml de dichlorométhane (3x). La phase organique est ensuite lavée successivement avec 250 ml de 3 M HCI, avec une solution de NaCI saturé et de NaH CO₃. Après séchage sur du MgSO₄, on filtre et concentre jusqu'à 400-500 ml. La solution visqueuse est ajoutée goutte à goutte à 1,5 litre de hexane sous forte agitation (mécanique de préférence). On agite ensuite encore durant 2 heures pour cristalliser le tout. Le solide est filtré, lavé à l'hexane et séché sous vide (0,2 mm Hg).
(2) Un mélange de 100 g de tosylate obtenu précédemment dans 500 ml de solution d'hydroxyde d'ammnonium (25 % NH₃ en H₂O) est chauffé dans un autoclave à l20°C durant 2 heures. Après refroidissement l'excès d'ammoniac est distillé sur un bain à l20-l30°C jusqu'à ce que la température de la vapeur atteigne 90°C. Après refroidissement on ajoute 30 ml de NaOH 2 M. On extrait au dichlorométhane et sèche sur MgSO₄. Après filtration et concentration le résidu est repris dans 500 ml de toluène. Cette solution est chauffée à 40°C, on y ajoute ensuite 150 ml d'isooctane. On refroidit à 5°C. Le précipité est filtré et lavé successivement au (1) toluène-isooctane (1:1) et (2) à l'isooctane. On sèche ensuite sous vide (0,2 mm Hg). On obtient ainsi 97 g de polymère aminé.

### Exemple 2

### Synthèse d'un conjugué PEG NH2 - CALCITONINE DE SAUMON

PEGNH₂ représente l'amino méthyl poly(oxyéthylène) de l'exemple 1
- Une solution de 30 µmoles de fmoc-CTS (le groupe fmoc protégeant les fonctions NH₂ de la CTS) dans 3 ml de DMF (diméthylformamide) est activée avec 300 µmoles de DCCI (dicyclohexylcarbodiimide) et avec 300 µmoles de NHSI (N-hydroxysuccinimide), pendant l heure, à température ambiante.
- On y ajoute ensuite 90 µmoles de méthyl poly(oxyéthylène) amine d'un poids moléculaire moyen de 5000, pendant 18 heures, à température ambiante.
- Après cette étape, 300 µl de diéthylamine y sont ajoutés, pendant 1 heure à température ambiante.
- Ensuite, on y ajoute 2 ml d'acide acétique 2 M., avant d'évaporer le tout sous vide.
- Le dérivé PEG NH2-CTS (PEG-NHCO-CTS) est alors isolé par HPLC, sur phase inverse C18.
- Il est à noter que la méthode décrite précédemment est applicable pour toute calciconine ou analogue ayant un groupe carboxylique libre en position 15.
Ainsi, un conjugué PEG NH2-Calcitonine humaine est également obtenu, par la méthode de couplage à l'acide aspartique en position 15 (en lieu et place de l'acide glutamique en position 15 dans la calcitonine de saumon).

### Exemple 3

### Reconnaissance du conjugué PEG NH2-CTS par les récepteurs membranaires

In vitro, le conjugué PEG NH2-CTS est toujours bien reconnu par les récepteurs membranaires isolés à partir de reins de rats selon la méthode décrite dans : "Methods in Enzymology, vol. 109, 1985".
Cette reconnaissance n'est plus valable avec un autre conjugué de polyéthylène glycol (PEG-COOH) et de CTS, où les PEG sont couplés sur les fonctions aminées libres de la calcitonine, selon une technique classiquement décrite pour le couplage PEG aux protéines. Le dérivé PEG-COOH utilisé était le méthoxypolyéthylène glycol-succinimidyl succinate (sigma M3152).
Ceci démontre bien l'importance de maintenir les fonctions amines libres des 2 lysines (ou arginine en cas de substitution de la lysine par de l'arginine) de la calcitonine de saumon pour l'intégrité de son activité biologique.

| | % de reconnaissance par récepteurs membranaires |
|---|---|
| CTS | 100 % |
| PEG NH2-CTS | 85 % |
| PEG COOH-CTS | < 0,5 % |

Les valeurs de pourcentage de reconnaisance par les récepteurs figurant dans ce tableau sont extrapolées comme suit, sur la courbe d'inhibition donnée en annexe I :
* une dilution de 1/200.000 du conjugué PEG-NH2-CTS devrait donner une valeur théorique de 3.5 ng/ml ; la valeur expérimentale est de 3 ng/ml = = 85 %.
* une dilution de 1/500 du conjugué PEG-COOH-CTS devrait donner une valeur théorique de 4 µg/ml ; la valeur expérimentale est de 6.5 ng/ml (< 0,5 %).

### Exemple 4

### Demi-vie plasmatique du conjugué PEG NH2-CTS

La calcitonine de saumon (35 µg/kg) et le dérivé PEG-NH2-CTS (35 µg/kg) ont été administrés en injection intraveineuse à des lapins.
Des échantillons sanguins sont ensuite prélevés régulièrement. Afin de déterminer la demi-vie plasmatique du conjugué PEG-NH2-CTS, les échantillons sériques sont dosés à l'aide d'un RIA ayant une sensibilité de 100 pg/tube.

| | Demi-vie plasmatique |
|---|---|
| CTS | 52 min. |
| PEG NH2-CTS | 130 min. |

Il est à noter que lors d'une injection intramusculaire la demi-vie plasmatique du confugué PEC NH₂-CTS est considérablement plus prolongée par rapport à la Calcitonine native en raison d'une résorption ralentie du conjugué ("effet dépôt").

### Exemple 5

### Masquage d'un épitope immunoréactif par le couplage d'une molécule de PEG en position 15 de la Calcitonine

Un conjugué de polyéthylène glycol aminé couplé sur l'acide aspartique de la calcitonine humaine a été synthétisé suivant la méthode décrite à l'exemple 2.
On dispose d'un dosage immunoradiométrique pour la Calcitonine humaine, utilisant 2 anticorps monoclonaux reconnaissant 2 épitopes différents sur la calcitonine humaine.
Par ce dosage, le dérivé PEG-NH2-CTH n'est plus reconnu qu'à 0.4 % par rapport à la calcitonine humaine.
Ceci signifie qu'au moins un épitope de la calcitonine humaine se trouve masqué par le couplage en position 15 d'une molécule de PEG.
Il y a donc une modification de l'antigénicité de la calcitonine, de par son couplage au PEG, qui diminue fortement sa reconnaissance par des anticorps.

| | CThumaine | PEG NH2-CThumaine |
|---|---|---|
| Concentration théorique : | 740µg/ml | 740µg/ml |
| Concentration dosée par l'IRMA : | 730µg/ml | 3µg/ml |

### Exemple 6

### Activité hypocalcémique du conjugué PEG-NH2-CTS

Selon le protocole décrit dans la pharmacopée européenne, des rats (OFA) ont été injectés en i.v. soit avec de la CTS, soit avec le conjugué PEG NH2-CTS synthétisé selon la méthode décrite à l'exemple 2, soit avec le conjugué PEG-COOH-CTS déjà utilisé dans l'exemple 3 (correspondant au couplage sur les amines).
Une hypocalcémie significative apparaît 1 heure après que les rats aient reçu une dose de couplage PEG-NH2-CTS identique à la dose contrôle de CTS (3 pM/rat, équivalent à 40 mUI pour la CTS).
Par contre, avec une même dose du conjugué PEG-COOH-CTS, aucune hypocalcémie n'est provoquée 1 heure après l'injection.

| Produit injecté en i.v. : | 40 mUI de CTS | 40 mUI de Peg-NH2-CTS | 40 mUI de Peg COOH -CTS |
|---|---|---|---|
| % de chute de la calcémie 1 heure après l'injection | 28 % | 24 % | 4 % (non significatif si<10 % ) |

### REFERENCES BIBLIOGRAPHIQUES

1. SCHWARTZ K.E., ORLOWSKI R.C., MARCUS R. :
   "des-Ser² Salmon Calcitonin : A biologically potent synthetic analog"
   Endocrinology 1981, 108/3, 831.
2. ORLOWSKI C.O., EPAND R.M., STAFFORD A.R. :
   "Biologically potent analogues of salmon calcitonin which do not contain an N-terminal disulfide-bridged ring structure"
   Eur. J. Biochem. 1987, 162, 399-402.
3. MAIER R., RINIKER B., RITTEL W. :
   "Analogues of human calcitonin. l) Influence of modifications in amino acid positions 29 and 31 on hypocalcaemic activity in the rat"
   Febs Letters 1974, 48/1, 68.
4. MORIKAWA T., MUNEKATA E., SAKAKIBARA S., NODA T., OTANI M. :
   "Synthesis of eel-calcitonin and (Asu¹7)-eel-calcitonin contribution of the disulfide bond to the hormonal activity"
   Experientia 1976, 32, 1104- 1106.
5. EPAND R.M., EPAND R.F., ORLOWSKI R.C., SEYLER J.K., COLESCOTT R.L. :
   Biochemistry 1986, 25, l964-l968.
6. RITTEL W., MAIER R., BRUGGER M., KAMBER B., RINIKER B., SIEBER P. :
   "Structure-activity relationship of human calcitonin.
   III. Biological activity of synthetic analogues with shortened or terminally modified peptide chains"
   Experientia 1976, 32, 246-248.
7. D'SANTOS C.S., NICHOLSON G.C., MOSELEY T., EVANS T, MARTIN T.J., KEMP B.E. :
   "Biologically active, derivatizable salmon calcitonin analog : design, synthesis and applications"
   Endocrinology 1988, l23/3, 1483.
8. ORLOWSKI R., HANAMURA S., MARUMOTO M., SAKAMOTO K., WAKI Y. :
   Patent CO7K7/l0, A61K37/02
   Int. Publication number : WO 90/12809
   Int. Publication date : 01 November 1990.
9. AZRIA M. :
   "The calcitonins : Physiology and Pharmacology"
   Ed. Karger 1989.
10. Calcitonin Series : Excerpta Medica Int Congr Ser :
   notamment :
   GUTTMAN S. :
   "Chemistry and structure-activity relationship of natural and synthetic calcitonins"
   Calcitonin 1980. Proc Int Symp, Milan 1980.
   Ed. PECILE A.
   Excerpta Medica Int Congr Ser 1981, 540, 11-24.
11. NEWSOME F.E., O'DOR R.K., PARKES C.O., COPP D.H. :
   "A study of the stability of calcitonin biological activity"
   Endocrinology 1973, 92, 1102-1106.
12. NUESCH E., SCHMIDT R. :
   "Comparative pharmacokinetics of calcitonins"
   Calcitonin 1980. Proc Int Symp, Milan 1980
   Ed. PECILE A.
   Excerpta Medica Int Congr Ser 1981, 540, 352-364.
13. ABUCHOWSKI A., VAN ES Th., PALCZUK N.C., DAVIS F.F. :
   "Alteration of Immunological properties of bovine serum albumin by covalent attachment of polyethylene glycol"
   J. Biol. Chem. 1977, 252/11, 3578-3581.
14. ABUCHOWSKI A., McCOY J.R., PALCZUK N.C., VAN ES Th., DAVIS F.F.:
   "Effect of covalent attachment of polyethylene glycol on immunogenicity and circulating life of bovine liver catalase"
   J. Biol. Chem. 1977, 252/11, 3582-3586.
15. ZALIFSKY S., GILON C., ZIEKHA A.
   Eur. Polymer. J. 1983, 19, 1177-1183.
16. MUTTER M. :
   Tetrah Lett 1978, 31, 1839.
17. JOHANSON I., GYSIN R., FLARNAGON S.D. :
   J. Biol. Chem. 1981, 256, 9126-9135.
18. BÜCKMANN, A.F.; MORR, M.; JOHANSSON, G. :
   Makromol. Chem. 1981, 182, 1379-1384.

## Revendications (Revendications pour l'(les) Etat(s) contractant(s) suivant(s): AT, BE, CH, LI, DE, DK, FR, GB, GR, IT, LU, MC, NL, PT, SE)

1. Conjugué de Calcitonine ou un polypeptide analogue avec un ou plusieurs composé(s) polymérique(s) comportant chacun un ou plusieurs polymère(s) de polyéthylène glycol par lien covalent du ou desdits composé(s) avec respectivement un ou au moins un groupe carboxyle de la Calcitonine ou son analogue.

2. Conjugué selon la revendication 2, caractérisé en ce que un dit composé polymérique est lié avec le groupe carboxyle d'un acide aminé acide en position 15 de la Calcitonine ou son analogue.

3. Conjugué de Calcitonine selon la revendication 1 ou 2, caractérisé en ce que ledit composé comporte une fonction amine libre et en ce que le lien covalent consiste en un lien amide -CONH- entre un dit groupe carboxyle de la Calcitonine ou son analogue et la fonction amine dudit composé.

4. Conjugué selon la revendication 3, caractérisé en ce que la fonction amine dudit composé est greffée directement sur un polymère de polyéthylène glycol, notamment à une de ses extrémités.

5. Conjugué selon la revendication 3, caractérisé en ce que la fonction dudit composé est portée par un groupe chimique consistant en un radical hydrocarboné linéaire ou cyclique dans lequel un CH₂ peut être remplacé par un hétéroatome S, N ou O, tel qu'un alkyl, un aryl, ou un hétérocycle, sur lequel est ou sont greffé(s) un ou plusieurs polymère(s) de polyéthylène glycol.

6. Conjugué selon la revendication 5, caractérisé en ce que ledit composé est un polyéthylène glycol homopolymère possédant d'une part un groupe NH₂ terminal à une extrémité, soit directement greffé, soit relié par un bras, et d'autre part un groupe OH terminal à l'autre extrémité qui est de préférence substitué avec un groupe alkyl de préférence méthyl.

7. Conjugué selon l'une des revendications 1 à 6, caractérisé en ce que ledit composé polymérique est l'α-amino-ω-méthylpoly(oxyéthylène).

8. Conjugué selon l'une des revendications 1 à 7, caractérisé en ce que ledit composé comprend un ou plusieurs poly(oxyéthylène) de poids moléculaire de 500 à 20 000.

9. Conjugué selon la revendication 8, caractérisé en ce que le poly(oxyéthylène) a un poids moléculaire de 4000 à 10 000.

10. Conjugué selon l'une des revendications 1 à 7, caractérisé en ce que la Calcitonine est une calcitonine humaine de saumon ou d'anguille ou un analogue avec un acide aspartique ou glutamique en position 15.

11. Procédé de préparation d'un conjugué selon l'une des revendications 1 à 9, caractérisé en ce que l'on effectue un couplage peptidique entre la fonction carboxyle de la Calcitonine ou d'un des ses analogues, et un groupe NH₂ d'un composé comportant un ou plusieurs polymère(s) de polyéthylène glycol, par un lien amide.

## Revendications (Revendications pour l'(les) Etat(s) contractant(s) suivant(s): ES)

1. Procédé de préparation d'un conjugué de Calcitonine ou d'un polypeptide analogue avec un ou plusieurs composé(s) polymérique(s) comportant chacun un ou plusieurs polymère(s) de polyéthylène glycol selon le(s)quel(s) on opère un lien covalent entre le ou lesdits composé(s) et respectivement un ou au moins un groupe carboxyle de la Calcitonine ou son analogue.

2. Procédé selon la revendication 1, caractérisé en ce que un dit composé polymérique est lié avec le groupe carboxyle d'un acide aminé acide en position 15 de la Calcitonine ou son analogue.

3. Procédé selon la revendication 1 ou 2, caractérisé en ce que ledit composé comporte une fonction amine libre et en ce que le lien covalent consiste en un lien amide -CONH- entre un dit groupe carboxyle de la Calcitonine ou son analogue et la fonction amine dudit composé.

4. Procédé selon la revendication 3, caractérisé en ce que la fonction amine dudit composé est greffée directement sur un polymère de polyéthylène glycol, notamment à une de ses extrémités.

5. Procédé selon la revendication 3, caractérisé en ce que la fonction dudit composé est portée par un groupe chimique consistant en un radical hydrocarboné linéaire ou cyclique dans lequel un CH₂ peut être remplacé par un hétéroatome S, N ou O, tel qu'un alkyl, un aryl, ou un hétérocycle, sur lequel est ou sont gréffé(s) un ou plusieurs polymère(s) de polyéthylène glycol.

6. Procédé selon la revendication 5, caractérisé en ce que ledit composé est un polyéthylène glycol homopolymère possédant d'une part un groupe NH₂ terminal à une extrémité, soit directement greffé, soit relié par un bras, et d'autre part un groupe OH terminal à l'autre extrémité qui est de préférence substitué avec un groupe alkyl de préférence méthyl.

7. Procédé selon l'une des revendications 1 à 6, caractérisé en ce que ledit composé polymèrique est l'α-amino-ω-méthylpoly(oxyéthylène).

8. Procédé selon l'une des revendications 1 à 7, caractérisé en ce que ledit composé comprend un ou plusieurs poly(oxyéthylène)de poids moléculaire de 500 à 20 000.

9. Procédé selon la revendication 8, caractérisé en ce que le poly(oxyéthylène) a un poids moléculaire de 4 000 à 10 000.

10. Procédé selon l'une des revendications 1 à 9, caractérisé en ce que la Calcitonine est une calcitonine humaine de saumon ou d'anguille ou un analogue avec un acide aspartique ou glutamique en position 15.

11. Procédé selon l'une des revendications 1 à 10 caractérisé en ce que l'on effectue un couplage peptidique entre la fonction carboxyle de la Calcitonine ou d'un de ses analogues, et un groupe NH₂ d'un composé comportant un ou plusieurs polymère(s) de polyéthylène glycol, par un lien amide.

## Patentansprüche (Patentansprüche für folgende(n) Vertragsstaat(en): AT, BE, CH, LI, DE, DK, FR, GB, GR, IT, LU, MC, NL, PT, SE)

1. Konjugat von Calcitonin oder einem analogen Polypeptid mit einer oder mehreren polymeren Verbindung(en), die jeweils ein oder mehrere Polymer(e) von Polyethylenglykol umfassen, durch kovalente Bindung des oder der Verbindung(en) mit jeweils einer oder mindestens einer Carboxylgruppe von Calcitonin oder seinem Analogon.

2. Konjugat nach Anspruch 1, dadurch gekennzeichnet , daß eine solche polymere Verbindung an die Carboxylgruppe einer sauren Aminosäure in Position 15 von Calcitonin oder seinem Analogon gebunden ist.

3. Konjugat von Calcitonin nach Anspruch 1 oder 2, dadurch gekennzeichnet , daß die Verbindung eine freie Aminfunktion umfaßt und daß die kovalente Bindung aus einer Amidbindung -CONH- zwischen einer Carboxylgruppe des Calcitonins oder seines Analogons und der Aminfunktion der Verbindung besteht.

4. Konjugat nach Anspruch 3, dadurch gekennzeichnet, daß die Aminfunktion der Verbindung direkt auf ein Polymer von Polyethylenglykol gepfropft ist, insbesondere auf eines seiner Enden.

5. Konjugat nach Anspruch 3, dadurch gekennzeichnet, daß die Funktion der Verbindung von einer chemischen Gruppe getragen wird, die aus einem linearen oder cyclischen Kohlenwasserstoffrest besteht, in dem ein CH₂ durch ein Heteroatom S, N oder O ersetzt sein kann, wie einem Alkyl, einem Aryl oder einem Heterocyclus, auf den ein oder mehrere Polymere von Polylethylenglykol gepfropft sind.

6. Konjugat nach Anspruch 5, dadurch gekennzeichnet , daß die Verbindung ein Polyethylenglykol-Homopolymer ist, das einerseits eine terminale NH₂-Gruppe an einem Ende, entweder direkt gepfropft oder durch einen Linker-Arm gebunden, und andererseits eine terminale OH-Gruppe am anderen Ende besitzt, die bevorzugt mit einer Alkylgruppe, bevorzugt mit Methyl substituiert ist.

7. Konjugat nach einem der Ansprüche 1 bis 6, dadurch gekennzeichnet , daß die polymere Verbindung α-Amino-ω-methylpoly(oxyethylen) ist.

8. Konjugat nach einem der Ansprüche 1 bis 7, dadurch gekennzeichnet , daß die Verbindung einen oder mehrere Poly(oxyethylen) mit einem Molekulargewicht von 500 bis 20000 umfaßt.

9. Konjugat nach Anspruch 8, dadurch gekennzeichnet , daß das Poly(oxyethylen) ein Molekulargewicht von 4000 bis 10000 hat.

10. Konjugat nach einem der Ansprüche 1 bis 7, dadurch gekennzeichnet , daß das Calcitonin ein Human Calcitonin, ein Calcitonin aus Lachs oder aus Aal oder ein Analogon mit einem Asparaginsäure- oder Glutaminsäurerest in Position 15 ist.

11. Verfahren zur Herstellung eines Konjugats nach einem der Ansprüche 1 bis 9, dadurch gekennzeichnet , daß man eine Peptidkopplung durch eine Amidbindung zwischen der Carboxylfunktion von Calcitonin oder einem seiner Analoga und einer NH₂-Gruppe einer Verbindung durchführt, die eine oder mehrere Polymere von Polyethylenglykol umfaßt.

## Patentansprüche (Patentansprüche für folgende(n) Vertragsstaat(en): ES)

1. Verfahren zur Herstellung eines Konjugats von Calcitonin oder eines analogen Polypeptids mit einer oder mehreren polymeren Verbindungen, die jeweils ein oder mehrere Polymer(e) von Polyethylenglykol umfassen, nach dem man eine kovalente Bindung zwischen der oder den Verbindung(en) und jeweils einer oder mindestens einer Carboxylgruppe des Calcitonins oder seines Analogons bildet.

2. Verfahren nach Anspruch 1, dadurch gekennzeichnet , daß eine solche polymere Verbindung an die Carboxylgruppe eines sauren Aminosäurerestes in Position 15 von Calcitonin oder seinem Analogon gebunden ist.

3. Verfahren nach Anspruch 1 oder 2, dadurch gekennzeichnet , daß die Verbindung eine freie Aminfunktion umfaßt und daß die kovalente Bindung aus einer Amidbindung -CONH- zwischen einer Carboxylgruppe des Calcitonins oder seines Analogons und der Aminfunktion der Verbindung besteht.

4. Verfahren nach Anspruch 3, dadurch gekennzeichnet , daß die Aminfunktion der Verbindung direkt auf ein Polymer von Polyethylenglykol gepfropft ist, insbesondere auf eines seiner Enden.

5. Verfahren nach Anspruch 3, dadurch gekennzeichnet , daß die Funktion der Verbindung von einer chemischen Gruppe getragen wird, die aus einem linearen oder cyclischen Kohlenwasserstoffrest besteht, in dem ein CH₂ durch ein Heteroatom S, N oder O ersetzt sein kann, wie einem Alkyl, einem Aryl oder einem Heterocyclus, auf den ein oder mehrere Polymere von Polylethylenglykol gepfropft sind.

6. Verfahren nach Anspruch 5, dadurch gekennzeichnet , daß die Verbindung ein Polyethylenglykol-Homopolymer ist, das einerseits eine terminale NH₂-Gruppe an einem Ende, entweder direkt gepfropft oder durch einen Linker-Arm gebunden, und andererseits eine terminale OH-Gruppe am anderen Ende besitzt, die bevorzugt mit einer Alkylgruppe, bevorzugt mit Methyl substituiert ist.

7. Verfahren nach einem der Ansprüche 1 bis 6, dadurch gekennzeichnet , daß die polymere Verbindung α-Amino-ω-methylpoly(oxyethylen) ist.

8. Verfahren nach einem der Ansprüche 1 bis 7, dadurch gekennzeichnet , daß die Verbindung einen oder mehrere Poly(oxyethylen) mit einem Molekulargewicht von 500 bis 20000 umfaßt.

9. Verfahren nach Anspruch 8, dadurch gekennzeichnet , daß das Poly(oxyethylen) ein Molekulargewicht von 4000 bis 10000 hat.

10. Verfahren nach einem der Ansprüche 1 bis 9, dadurch gekennzeichnet , daß das Calcitonin ein Human Calcitonin, ein Calcitonin aus Lachs oder aus Aal oder ein Analogon mit einem Asparaginsäure- oder Glutaminsäurerest in Position 15 ist.

11. Verfahren zur Herstellung eines Konjugats nach einem der Ansprüche 1 bis 10, dadurch gekennzeichnet , daß man eine Peptidkopplung durch eine Amidbindung zwischen der Carboxylfunktion von Calcitonin oder einem seiner Analoga und einer NH₂-Gruppe einer Verbindung durchführt, die ein oder mehrere Polymer(e) von Polyethylenglykol umfaßt.

## Claims (Claims for the following Contracting State(s): AT, BE, CH, LI, DE, DK, FR, GB, GR, IT, LU, MC, NL, PT, SE)

1. Conjugate of calcitonin or an analogous polypeptide with one or more polymetric compound(s) each comprising one or more polyethylene glycol polymer(s) by covalent bonding of the said compound(s) with respectively one or at least one carboxyl group of the calcitonin or its analogue.

2. Conjugate according to Claim 1, characterized in that a said polymeric compound is bonded with the carboxyl group of an acidic amino acid in the 15-position of the calcitonin or its analogue.

3. Calcitonin conjugate according to Claim 1 or 2, characterized in that the said compound comprises a free amine functional group and in that the covalent bond consists of an amide bond -CONH- between a said carboxyl group of the calcitonin or its analogue and the amine functional group of the said compound.

4. Conjugate according to Claim 3, characterized in that the amine functional group of the said compound is grafted directly onto a polyethylene glycol polymer, in particular at one of its ends.

5. Conjugate according to Claim 3, characterized in that the functional group of the said compound is carried by a chemical group consisting of a linear or cyclic hydrocarbon radical in which a CH₂ can be replaced by an S, N or O heteroatom, such as an alkyl, an aryl or a heterocycle, on which is or are grafted one or more polyethylene glycol polymer(s).

6. Conjugate according to Claim 5, characterized in that the said compound is a polyethylene glycol homopolymer possessing, on the one hand, a terminal NH₂ group at one end, either directly grafted or connected via an arm, and, on the other hand, a terminal OH group at the other end which is preferably substituted with an alkyl group, preferably a methyl group.

7. Conjugate according to one of Claims 1 to 6, characterized in that the said polymeric compound is α-amino-ω-methylpoly(oxyethylene).

8. Conjugate according to one of Claims 1 to 7, characterized in that the said compound comprises one or more poly(oxyethylene)(s) with a molecular weight of 500 to 20,000.

9. Conjugate according to Claim 8, characterized in that the poly(oxyethylene) has a molecular weight of 4000 to 10,000.

10. Conjugate according to one of Claims 1 to 7, characterized in that the calcitonin is a human, salmon or eel calcitonin or an analogue with an aspartic or glutamic acid in the 15-position.

11. Process for the preparation of a conjugate according to one of Claims 1 to 9, characterized in that a peptide coupling is carried out, via an amide bond, between the carboxyl functional group of the calcitonin or of one of its analogues and an NH₂ group of a compound comprising one or more polyethylene glycol polymer(s).

## Claims (Claims for the following Contracting State(s): ES)

1. Process for the preparation of a conjugate of calcitonin or of an analogous polypeptide with one or more polymeric compound(s) each comprising one or more polyethylene glycol polymer(s), according to which a covalent bond is produced between the said compound(s) and respectively one or at least one carboxyl group of the calcitonin or its analogue.

2. Process according to Claim 1, characterized in that a said polymeric compound is bonded with the carboxyl group of an acidic amino acid in the 15-position of the calcitonin or its analogue.

3. Process according to Claim 1 or 2, characterized in that the said compound comprises a free amine functional group and in that the covalent bond consists of an amide bond -CONH- between a said carboxyl group of the calcitonin or its analogue and the amine functional group of the said compound.

4. Process according to Claim 3, characterized in that the amine functional group of the said compound is grafted directly onto a polyethylene glycol polymer, in particular at one of its ends.

5. Process according to Claim 3, characterized in that the functional group of the said compound is carried by a chemical group consisting of a linear or cyclic hydrocarbon radical in which a CH₂ can be replaced by an S, N or O heteroatom, such as an alkyl, an aryl or a heterocycle, on which is or are grafted one or more polyethylene glycol polymer(s).

6. Process according to Claim 5, characterized in that the said compound is a polyethylene glycol homopolymer possessing, on the one hand, a terminal NH₂ group at one end, either directly grafted or connected via an arm, and, on the other hand, a terminal OH group at the other end which is preferably substituted with an alkyl group, preferably a methyl group.

7. Process according to one of Claims 1 to 6, characterized in that the said polymeric compound is α-amino-ω-methylpoly(oxyethylene).

8. Process according to one of Claims 1 to 7, characterized in that the said compound comprises one or more poly(oxyethylene)(s) with a molecular weight of 500 to 20,000.

9. Process according to Claim 8, characterized in that the poly(oxyethylene) has a molecular weight of 4000 to 10,000.

10. Process according to one of Claims 1 to 9, characterized in that the calcitonin is a human, salmon or eel calcitonin or an analogue with an aspartic or glutamic acid in the 15-position.

11. Process according to one of Claims 1 to 10, characterized in that a peptide coupling is carried out, via an amide bond, between the carboxyl functional group of the calcitonin or of one of its analogues and an NH₂ group of a compound comprising one or more polyethylene glycol polymer(s).
